(19) Europäisches Patentamt
European Patent Office
Office européen des brevets

(11) Veröffentlichungsnummer: **0 525 568 B1**

# (12) EUROPÄISCHE PATENTSCHRIFT

(45) Veröffentlichungstag der Patentschrift: **10.05.95**

(51) Int. Cl.[6]: **C07D 211/90**, A61K 31/44

(21) Anmeldenummer: **92112355.0**

(22) Anmeldetag: **20.07.92**

Die Akte enthält technische Angaben, die nach dem Eingang der Anmeldung eingereicht wurden und die nicht in dieser Patentschrift enthalten sind.

## (54) N-alkylierte 1,4-Dihydropyridincarbonsäureester.

(30) Priorität: **31.07.91 DE 4125271**

(43) Veröffentlichungstag der Anmeldung:
**03.02.93 Patentblatt 93/05**

(45) Bekanntmachung des Hinweises auf die Patenterteilung:
**10.05.95 Patentblatt 95/19**

(84) Benannte Vertragsstaaten:
**AT BE CH DE DK ES FR GB GR IT LI LU NL PT SE**

(56) Entgegenhaltungen:
**EP-A- 0 322 747**
**EP-A- 0 451 654**

**CHEMICAL ABSTRACTS, vol. 110, no. 11, 13. März 1989, Columbus, Ohio, US;abstract no. 95014J, 'preparation of optically active dihydropyridinecarboxylic acid esters as cardiovascular agents' Seite 686 ;**

(73) Patentinhaber: **BAYER AG**

**D-51368 Leverkusen (DE)**

(72) Erfinder: **Rosen, Bruno, Dr.**
**Marienburger Strasse 65**
**W-5603 Wülfrath (DE)**
Erfinder: **Zaiss, Siegfried, Dr.**
**Farnweg 3**
**W-5600 Wuppertal (DE)**
Erfinder: **Wollweber, Hartmund, Dr.**
**In den Birken 73**
**W-5600 Wuppertal 1 (DE)**
Erfinder: **Dellweg, Hans-Georg, Dr.**
**In den Birken 46**
**W-5600 Wuppertal 1 (DE)**
Erfinder: **de Jonge, Maarten, Dr.**
**Am Kreuzberg 9**
**W-5063 Overath-Steinenbrück (DE)**



Anmerkung: Innerhalb von neun Monaten nach der Bekanntmachung des Hinweises auf die Erteilung des europäischen Patents kann jedermann beim Europäischen Patentamt gegen das erteilte europäische Patent Einspruch einlegen. Der Einspruch ist schriftlich einzureichen und zu begründen. Er gilt erst als eingelegt, wenn die Einspruchsgebühr entrichtet worden ist (Art. 99(1) Europäisches Patentübereinkommen).

**CHEMICAL ABSTRACTS, vol. 114, no. 25, 24. Juni 1991, Columbus, Ohio, US;abstract no. 247146B, 'preparation of phenyldihydropyridine derivatives ascalcium antagonists and antihypertensives' Seite 748 ;**

**TRENDS PHARMACOL. SCI. Bd. 11, Nr. 8, 1990, Seiten 309 - 310; 'nimodipine andthe recovery of memory'**

**PHYSIOL. BEHAV. Bd. 42, Nr. 5, 1988, Seiten 447 - 452; 'the effect of a calciumantagonist on the retention of simple associational learning'**

## Beschreibung

Die Erfindung betrifft neue N-alkylierte 1,4-Dihydropyridindicarbonsäureester, Verfahren zu ihrer Herstellung und ihre Verwendung in Arzneimitteln, insbesondere als Cerebral-Therapeutika bei neuronalen Ausfallerscheinungen.

Es ist bereits bekannt, daß 1,4-Dihydropyridindicarbonsäureester eine calciumantagonistische oder calciumagonistische Wirkkung besitzen und somit als kreislaufbeeinflussende Mittel eingesetzt werden können [vgl. z. B. DOS 25 06 987; DOS 22 10 667; EP 240 828].

In EP-A-0 451 654, die nach dem Prioritätstag der vorliegenden Anmeldung publiziert wurde, werden ähnliche Dihydropyridine mit cerebraler Wirkung beschrieben, die sich jedoch formal eindeutig von den beanspruchten Verbindungen unterscheiden. In C. A., Bd. 110, 95014j und C. A., Bd. 114, 247146b und auch in EP-A-0 322 747 werden Dihydropyridine mit calciumantagonistischer Wirkung beschrieben, die sich in ihrer chemischen Struktur und in ihrem Wirkungsprofil eindeutig von den beanspruchten Verbindungen unterscheiden. Als typischer Vertreter eines cerebral wirksamen Dihydropyridins ist Nimodipin bekannt (vgl. Trends Pharmacol. Science, Bd. 11, 1990, Seiten 309-310 und Physiol. Behav., Bd. 42, 1988, Seiten 447-452). Die cerebrale Wirkung von Nimodipin und entsprechenden bekannten Dihydropyridinen beruht im wesentlichen auf der calciumantagonistischen Wirkung und auf ihrer starken Bindung an DHP-Rezeptoren des Calcium-L-Kanal in Gehirn- und Nervenzellen.

Die Erfindung betrifft N-alkylierte 1,4-Dihydropyridindicarbonsäureester der allgemeinen Formel (I)

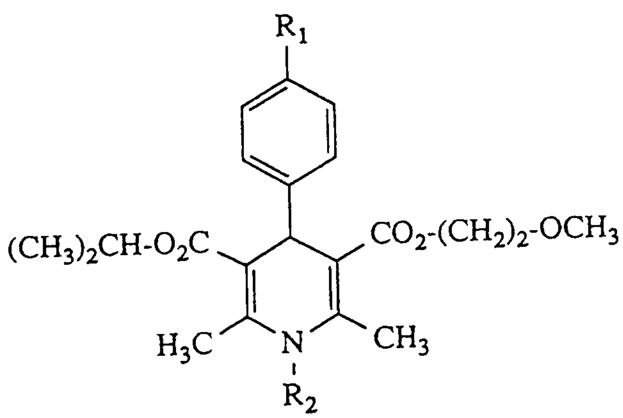

(I)

in welcher

$R^1$ für Trifluormethoxy, Cyano, Fluor, Chlor oder Trifluormethyl steht,

$R^2$ für Methyl, Ethyl oder Cyclopropyl steht,

mit der Maßgabe, daß $R^1$ nicht für Trifluormethyl stehen darf, wenn $R^2$ Methyl bedeutet.

Die erfindungsgemäßen Verbindungen können in stereoisomeren Formen existieren, die sich entweder wie Bild und Spiegelbild (Enantiomere) oder nicht als Bild und Spiegelbild (Diastereomere), verhalten. Die Erfindung betrifft sowohl die Antipoden als auch die Racemformen. Die Racemformen lassen sich in bekannter Weise in die stereoisomer einheitlichen Bestandteile trennen [vgl. E.L. Eliel, Stereochemistry of Carbon Compounds, McGraw Hill, 1962].

Bevorzugt sind folgende Verbindungen der allgemeinen Formel (I):

1,2,6-Trimethyl-4-(4-trifluormethoxyphenyl)-1,4-dihydropyridin-3,5-dicarbonsäure-isopropyl-(2-methoxyethyl)-ester,

1,2,6-Trimethyl-4-(4-cyanophenyl)-1,4-dihydropyridin-3,5-dicarbonsäureisopropyl-(2-methoxyethyl)-ester,

1-Ethyl-2,6-dimethyl-4-(4-trifluormethoxyphenyl)-1,4-dihydropyridin-3,5-dicarbonsäure-isopropyl-(2-methoxyethyl)-ester,

1,2,6-Trimethyl-4-(4-chlorphenyl)-1,4-dihydropyridin-3,5-dicarbonsäure-isopropyl-(2-methoxyethyl)-ester,

1,2,6-Trimethyl-4-(4-fluorphenyl)-1,4-dihydropyridin-3,5-dicarbonsäure-isopropyl-(2-methoxyethyl)-ester,

1-Ethyl-2,6-dimethyl-4-(4-trifluormethylphenyl)-1,4-dihydropyndin-3,5-dicarbonsäure-isopropyl-(2-methoxyethyl)-ester,

1-Cyclopropyl-2,6-dimethyl-4-(4-trifluormethoxyphenyl)-1,4-dihydropyridin-3,5-dicarbonsäure-isopropyl-(2-methoxyethyl)-ester, in Form ihrer Racemate, (+)- oder (-)-Isomeren.

Besonders bevorzugt sind folgende Verbindungen der allgemeinen Formel (I):

(±)-1,2,6-Trimethyl-4-(4-trifluormethoxyphenyl)-1,4-dihydropyridin-3,5-dicarbonsäure-isopropyl-(2-methoxyethyl)-ester,

(+)-1,2,6-Trimethyl-4-(4-trifluormethoxyphenyl)-1,4-dihydropyridin-3,5-dicarbonsäure-isopropyl-(2-methoxyethyl)-ester,
(-)-1,2,6-Trimethyl-4-(4-trifluormethoxyphenyl)-1,4-dihydropyridin-3,5-dicarbonsäure-isopropyl-(2-methoxyethyl)-ester,
(±)-1,2,6-Trimethyl-4-(4-chlorphenyl)-1,4-dihydropyridin-3,5-dicarbonsäure-isopropyl-(2-methoxyethyl)-ester,
(+)-1,2,6-Trimethyl-4-(4-chlorphenyl)-1,4-dihydropyridin-3,5-dicarbonsäure-isopropyl-(2-methoxyethyl)-ester,
(-)-1,2,6-Trimethyl-4-(4-chlorphenyl)-1,4-dihydropyridin-3,5-dicarbonsäure-isopropyl-(2-methoxyethyl)-ester.

Ganz besonders bevorzugt sind die folgenden Verbindungen der allgemeinen Formel (I):
(±)-1,2,6-Trimethyl-4-(4-trifluormethoxyphenyl)-1,4-dihydropyridin-3,5-dicarbonsäure-isopropyl-(2-methoxyethyl)-ester,
(+)-1,2,6-Trimethyl-4-(4-trifluormethoxyphenyl)-1,4-dihydropyridin-3,5-dicarbonsäure-isopropyl-(2-methoxyethyl)-ester,
(-)-1,2,6-Trimethyl-4-(4-trifluormethoxyphenyl)-1,4-dihydropyridin-3,5-dicarbonsäure-isopropyl-(2-methoxyethyl)-ester.

Die erfindungsgemäßen Verbindungen der allgemeinen Formel (I) können hergestellt werden, indem man

[A] Aldehyde der allgemeinen Formel (II)

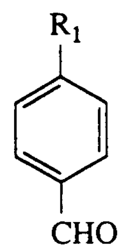

(II)

in welcher

$R^1$ die oben angegebene Bedeutung hat,

entweder zuerst mit Acetessigsäureisopropylester der Formel (III) oder mit Acetessigsäure-2-methoxyethylester der Formel (IV)

$(CH_3)_2CH\text{-}O_2C\text{-}CH_2\text{-}CO\text{-}CH_3$ (III)

$H_3C\text{-}CO\text{-}CH_2\text{-}CO_2\text{-}(CH_2)_2\text{-}OCH_3$ (IV)

gegebenenfalls unter Isolierung, in die entsprechenden Ylidenverbindungen der allgemeinen Formel (V) oder (VI)

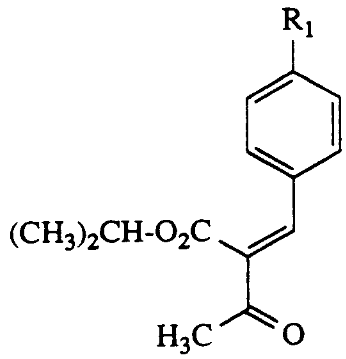

(V)

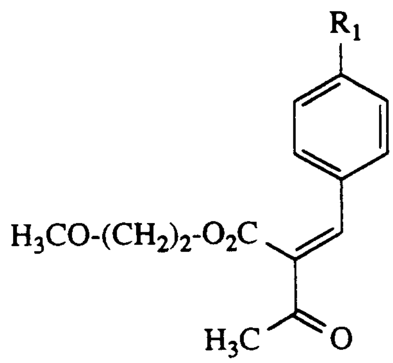

(VI)

in welcher

$R^1$ die oben angegebene Bedeutung hat,

überführt, anschließend im Fall der Verbindungen der allgemeinen Formel (V) mit Acetessigsäure-2-methoxyethylester der Formel (IV) und im Fall der Verbindungen der allgemeinen Formel (VI) mit Acetessigsäureisopropylester der Formel (III),

und mit Aminen oder den entsprechenden Aminhydrochloriden der allgemeinen Formel (VII)

$H_2N\text{-}R^2$ (VII)

in welcher

$R^2$ die oben angegebene Bedeutung hat,

in inerten Lösemitteln umsetzt,

oder

[B] im Fall, daß $R^2$ für Methyl oder Ethyl steht,

zunächst die Verbindungen der allgemeinen Formel (V) mit Aminocrotonsäure-2-methoxyethylester der Formel (VIII)

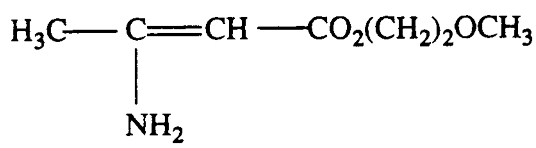

(VIII)

oder die Verbindungen der allgemeinen Formel (VI) mit $\beta$-Aminocrotonsäure-2-isopropylester der Formel (IX)

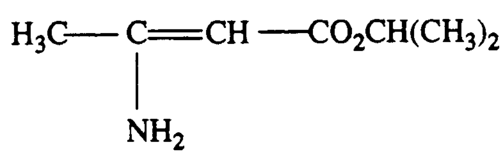

(IX)

in inerten Lösemitteln umsetzt und in einem letzten Schritt nach üblicher Methode, gegebenenfalls in Anwesenheit einer Base die -NH-Funktion alkyliert
und im Fall der enantiomerenreinen Ester entweder direkt eine chromatographische Trennung anschließt oder gegebenenfalls die jeweiligen enantiomerenreinen Carbonsäuren herstellt und diese, gegebenenfalls über ein reaktives Säurederivat, nach üblicher Methode verestert.

Beispielsweise soll das Verfahren [A] durch folgendes Formelschema erläutert werden:

[A]

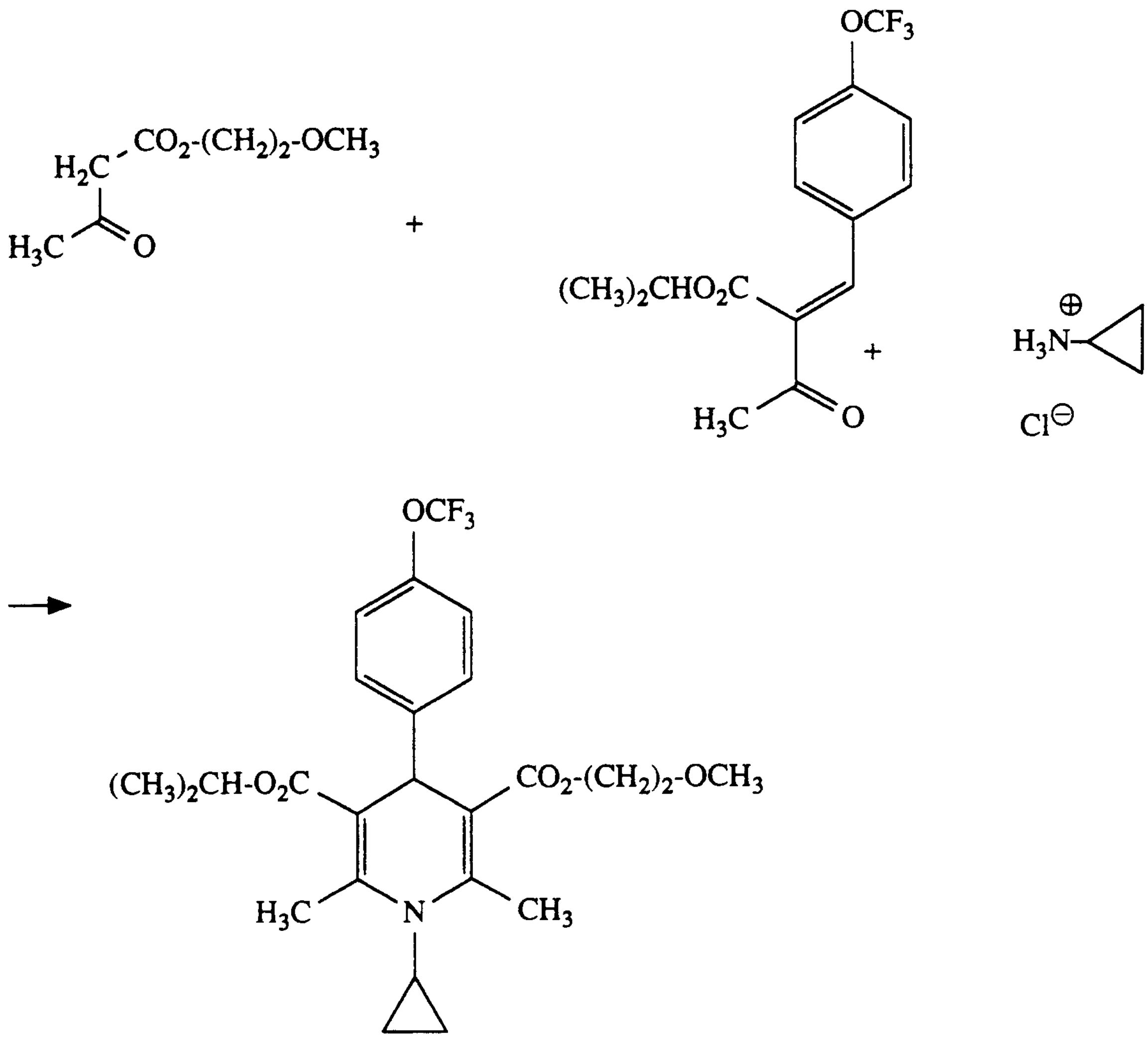

Als Lösemittel kommen Wasser oder organische Lösemittel in Frage, die sich unter den Reaktionsbedingungen nicht verändern. Hierzu gehören vorzugsweise Alkohole wie Methanol, Ethanol, Propanol oder Isopropanol, Ether wie 1,2-Dimethoxyethan, Diethylether, Dioxan, Tetrahydrofuran, Glykolmonomethylether oder Glykoldimethylether, oder Amide wie Dimethylformamid, Dimethylacetamid oder Hexamethylphosphorsäuretriamid, oder Eisessig, Dimethylsulfoxid, Acetonitril oder Pyridin. Bevorzugt sind Pyridin und 1,2-Dimethoxyethan.

Als Basen eignen sich im allgemeinen Alkalihydride wie Kalium- oder Natriumhydrid, oder Alkalialkoholate wie Natrium- oder Kaliummethanolat, Natrium- oder Kaliumethanolat oder Kalium-tert.butylat. Bevorzugt ist Natriumhydrid.

Die Base wird in einer Menge von 1,0 mol bis 1,5 mol, bevorzugt von 1,0 mol bis 1,3 mol bezogen auf 1 mol des Dihydropyridins eingesetzt.

Die Reaktionstemperaturen können in einem größeren Bereich variiert werden. Im allgemeinen arbeitet man zwischen +10 °C und +150 °C, vorzugsweise zwischen +20 °C und +100 °C. Insbesondere bei der Siedetemperatur des jeweiligen Lösemittels.

Die Umsetzung kann bei Normaldruck, aber auch bei erhöhtem oder erniedrigtem Druck durchgeführt werden. Im allgemeinen arbeitet man bei Normaldruck.

Bei der Durchführung der erfindungsgemäßen Verfahrensvarianten A und B ist das Verhältnis der an der Reaktion beteiligten Stoffe beliebig. Im allgemeinen arbeitet man jedoch mit molaren Mengen der Reaktanden. Die Isolierung und Reinigung der erfindungsgemäßen Substanzen erfolgt vorzugsweise derart, daß man das Lösemittel im Vakuum abdestilliert und den gegebenenfalls erst nach Eiskühlung kristallin erhaltenen Rückstand aus einem geeigneten Lösemittel umkristallisiert. In einigen Fällen kann es erforderlich sein, die erfindungsgemäßen Verbindungen durch Chromatographie zu reinigen.

Die Aldehyde der allgemeinen Formel (II) sind bekannt oder können nach üblicher Methode hergestellt werden [vgl. DOS 21 65 260; 24 01 665; T.D. Harris, G.P. Roth, J. Org. Chem. 44, 2004 (1979); W.J. Dale, H.E. Hennis, J. Am. Chem. Soc. 78, 2543 (1956); Chem. Abstr. 59, 13929 (1963)].

Die Verbindungen der allgemeinen Formeln (III), (IV), (VIII) und (IX) sind bekannt [vgl. z.B. MSD Book 2, 22506; Beilstein 3,632; 3,654].

Die Ylidenverbindungen der allgemeinen Formeln (V) und (VI) sind bekannt oder können nach üblicher Methode hergestellt werden [vgl. Lieb. Ann. Chem. 602, 14 (1957)].

Die Verbindungen der allgemeinen Formel (VII) sind bekannt [vgl. Beil. 4, 87 und 12, 3].

Als Alkylierungsmittel bei dem Verfahren können beispielsweise Alkylhalogenide, Sulfonsäureester oder substituierte oder unsubstituierte Dialkylsulfate, vorzugsweise Alkyliodide oder p-Toluolsulfonsäureester eingesetzt werden.

Die Alkylierung wird in den oben aufgeführten Lösemitteln bei Temperaturen von 0°C bis +150°C, vorzugsweise bei +20°C bis +100°C bei Normaldruck durchgeführt.

Im Fall der Herstellung der enantiomerenreinen Ester der allgemeinen Formel (I) über die entsprechenden enantiomerenreinen Säuren eignen sich als aktivierende Reagenzien zur Herstellung der reaktiven Säurederivate vor der Veresterung neben den anorganischen Halogeniden wie Thionylchlorid, Phosphortrichlorid oder Phosphorpentachlorid, oder dem Carbonyldiimidazol, Carbodiimide wie Cyclohexylcarbodiimid oder 1-Cyclohexyl-3-[2-(N-methyl-morpholino)ethyl]carbodiimid-p-toluolsulfonat oder N-Hydroxyphthalimid oder N-Hydroxy-benztriazol in Gegenwart von Dicyclohexylcarbodiimid.

Als Lösemittel für die Umsetzung mit den entsprechenden Alkoholen eignen sich die oben aufgeführten Lösemittel mit Ausnahme der Alkohole.

Bevorzugt erfolgt die Darstellung der enantiomerenreinen Verbindungen der allgemeinen Formel (I) über eine chromatographische Trennung an chiralen Säulen nach üblicher Methode.

Die neuen erfindungsgemäßen Verbindungen zeigen ein nicht vorhersehbares, wertvolles pharmakologisches Wirkspektrum.

Verbunden mit einem blutdruckneutralen Verhalten in einem Dosis-Bereich bis mindestens 10 mg/kg i.v. und ≦ 100 mg/kg p.o. beeinflussen sie als Cerebraltherapeutika neuronale Faktoren positiv.

Sie können deshalb für die Herstellung von Arzneimitteln zur Prophylaxe und Behandlung von neuronalen Ausfallerscheinungen, auch solchen, die auf der Basis von Durchblutungsstörungen entstehen, eingesetzt werden. Dazu gehören die Beseitigung kognitiver Defizite, die Verbesserung von Lern- und Gedächtnisleistungen sowie die Behandlung der Alzheimer'schen Krankheit.

Passiver Vermeidungstest

Eine Woche vor Testbeginn wurden die Ratten in eine geräuschundurchdringliche Kammer, in der alle Tests vorgenommen werden, gebracht. Während des Versuchdurchgangs 1 (t=oh) wurde jedes Tier auf eine Kleine, schwarze Plattform gesetzt, die durch eine 60 W starke Glühbirne helle erleuchtet war. Die Plattform hatte einen Zugang zu einem dunklen Abschnitt mit einem Gitterboden. Die Latenz bis zum Betreten des dunklen Abschnitts wurde gemessen. Dieser Test wurde nach 4 Stunden ein zweites Mal unter gleichen Bedingungen durchgeführt. Dadurch wurden die Tiere an die Testsituation gewöhnt.

Während des 3. Versuchdurchgangs nach 24h, dem Schockversuch, wurden die Tiere beim Betreten des dunklen Abschnitts für 2 sec. einem 220 µA starken elektrischen Schock an den Füßen ausgesetzt.

30 min. vor dem Schockversuch wurden entweder die erfindungsgemäßen Verbindungen (3 Gruppen mit jeweils 10 Tieren) oder Placebo in der jeweiligen Kontrollgruppe mit 10 Tieren oral (1 % Tylose) verabreicht.

25h nach dem Schockversuch (4. Versuchdurchgang, Reaktionsversuch t=48h) wurde der Versuchdurchgang 1 wiederholt.

Für alle Versuchdurchgänge wurde eine maximale Latenz von 180 sec. erlaubt.

Die Differenzen in den Latenzwerten zwischen den Kontroll- und Experiment-Gruppen, die beim Verhaltensversuch aufgetreten sind wurden nach den Mann-Whitney U-Tests und einer Varianzanalyse

statistisch evaluiert.

Unterschiede in der Anzahl der Tiere, die den dunklen Abschnitt nicht betreten haben (maximal avoidance latencies), wurden unter Verwendung dem Fisher Exact Probabilitytest statistisch bewertet.

Wenn im Reaktionsversuch (4. Versuchsdurchgang) die Tieren, denen die erfindungsgemäßen Verbindungen verabreicht wurden, im Gegensatz zu den Placebo-Tieren, entweder längere Zeit bis zum Betreten des dunklen Abschnitts benötigten oder diesen überhaupt nicht betraten so impliziert dies, daß die Tiere unter Einfluß der erfindungsgemäßen Verbindungen eine bessere Erinnerung an den Schock-Versuch 23h zuvor besitzen.

Latenzwerte in Sekunden

Beispiel 10

| | Versuchsdurchgang | Versuchsdurchgang | Versuchsdurchgang (Schock) | Versuchsdurchgang (Retention) |
|---|---|---|---|---|
| Placebo | 14,4(2,23) | 6,9(0,74) | 66(1,34) | 126,4(22,72) |
| 2,5 | 26,8(11,35) | 9,6(1,83) | 7,9(1,99) | 162,4(12,84) |
| 5,0 | 15,6(2,28) | 7,4(1,64) | 6,0(1,15) | 170,9(6,44) |
| 10,0 | 15,6(2,83) | 11,4(4,91) | 3,7(0,62) | 180,0(0,00) |

Zur vorliegenden Erfindung gehören pharmazeutische Zubereitungen, die neben nicht-toxischen, inerten pharmazeutisch geeigneten Trägerstoffen die erfindungsgemäßen Verbindungen enthalten, sowie Verfahren zur Herstellung dieser Zubereitungen.

Die oben aufgeführten pharmazeutischen Zubereitungen können außer den erfindungsgemäßen Verbindungen auch weitere pharmazeutische Wirkstoffe enthalten.

Die Herstellung der oben aufgeführten pharmazeutischen Zubereitungen erfolgt in üblicher Weise nach bekannten Methoden, z.B. durch Mischen des oder der Wirkstoffe mit dem oder den Trägerstoffen.

Im allgemeinen hat es sich als vorteilhaft erwiesen, den oder die erfindungsgemäßen Wirkstoffe in Gesamtmengen von etwa 0,5 bis etwa 500, vorzugsweise 1 bis 100 mg/kg Körpergewicht je 24 Stunden, gegebenenfalls in Form mehrerer Einzelgaben, zur Erzielung der gewünschten Ergebnisse zu verabreichen. Eine Einzelgabe enthält den oder die Wirkstoffe vorzugsweise in Mengen von etwa 1 bis etwa 80, insbesondere 1 bis 30 mg/kg Körpergewicht. Es kann jedoch erforderlich sein, von den genannten Dosierungen abzuweichen, und zwar in Abhängigkeit von der Art und dem Körpergewicht des zu behandelnden Objekts, der Art und der Schwere der Erkrankung, der Art der Zubereitung und der Applikation des Arzneimittels sowie dem Zeitraum bzw. Intervall, innerhalb welchem die Verabreichung erfolgt.

Herstellungsbeispiele

Beispiel 1

(+)-4-(4-Chlorphenyl)-1,2,6-trimethyl-1,4-dihydropyndin-3,5-dicarbonsäureisopropyl-2-methoxyethylester

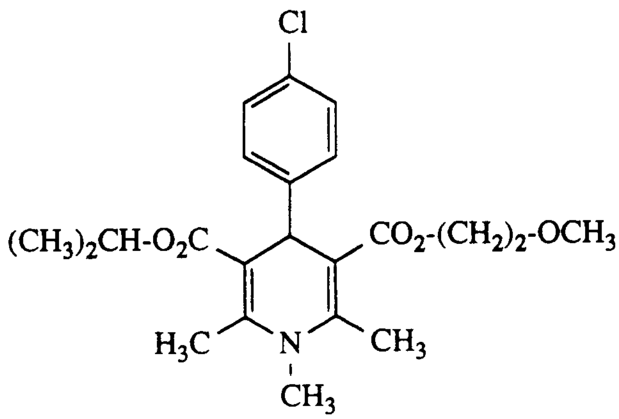

3,5 g (8,6 mmol) (+)-4-(4-Chlorphenyl)-2,6-dimethyl-1,4-dihydropyridin-3,5-dicarbonsäureisopropyl-2-methoxyethylester werden in 40 ml 1,2-Dimethoxyethan gelöst, bei 0°C mit 310 mg Natriumhydrid (80%ig in Weißöl, ca. 10,3 mmol) versetzt und 30 min bei dieser Temperatur gerührt. Danach fügt man 0,65 ml (10,3 mmol) Jodmethan zu und rührt 1 h bei 0°C, 20 h bei Zimmertemperatur. Man stellt das Reaktionsgemisch mit Eisessig schwach sauer, engt im Vakuum ein und nimmt den Rückstand in Dichlormethan auf. Nach Waschen mit Wasser, Trocknen über Natriumsulfat und Einengen reinigt man das resultierende Rohprodukt durch Chromatographie an Kieselgel im Laufmittel Dichlormethan / Essigester 20:1 und erhält so 2,6 g (72% der Theorie) der Titelverbindung.
$[\alpha]_D^{20}$ = +13,7 (c = 0,907, $CHCl_3$)
In Analogie zur Vorschrift des Beispiels 1 werden die in Tabelle 1 aufgeführten Beispiele hergestellt:

Tabelle 1:

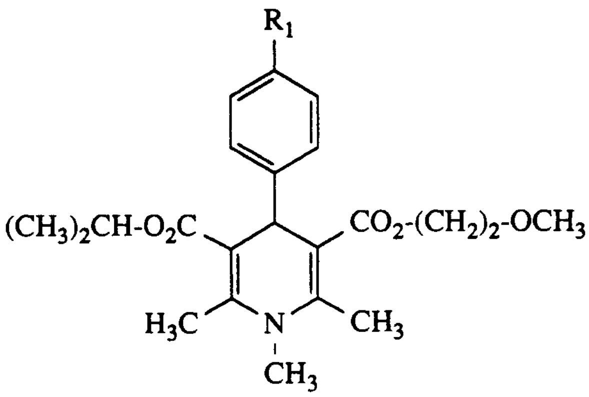

| Bsp.-Nr. | $R^1$ | Ausbeute (% d.Th.) | F°C | $[\alpha]_D^{20}$ |
|---|---|---|---|---|
| 2 | -Cl | 54 | Öl | -7,8 (c=1,08, $CHCl_3$)<br>(-)-Enantiomer |
| 3 | -Cl | 64 | Öl | Racemat |
| 4 | -F | 55 | Öl | Racemat |
| 5 | -CN | 54 | Öl | Racemat |
| 6 | -F | 60 | Öl | -15,7 ($CHCl_3$)<br>(-)-Enantiomer |
| 7 | -F | 63 | Öl | +18,9 ($CHCl_3$)<br>(+)-Enantiomer |
| 8 | $-OCF_3$ | 50 | Öl | -12,7<br>(-)-Enantiomer |
| 9 | $-OCF_3$ | 68 | Öl | -11,4 ($CHCl_3$)<br>(+)-Enantiomer |
| 10 | $-OCF_3$ | 60 | 45-47 | Racemat |

Beispiel 11

1-Cyclopropyl-2,6-dimethyl-4-(4-trifluormethoxyphenyl)-1,4-dihydropyridin-3,5-dicarbonsäure-isopropyl-2-methoxyethylester

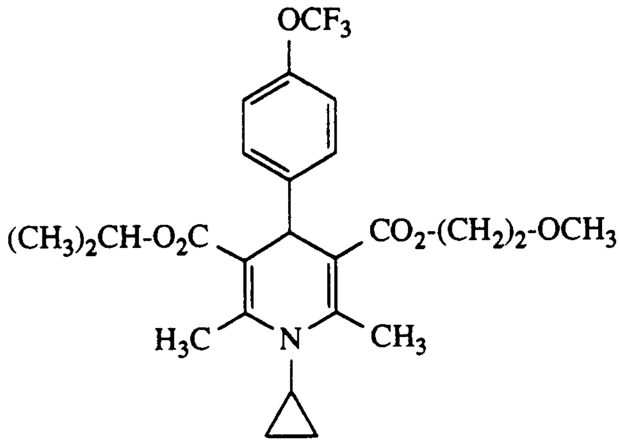

16,4 g (52 mmol) 2-Acetyl-4-(4-trifluormethoxyphenyl)-3-butensäureisopropylester, 8,3 g (52 mmol) Acetessigsäure-2-methoxyethylester und 6,3 g (67 mmol) Cyclopropylaminhydrochlorid werden in 60 ml Pyridin 5 h zum Rückfluß erhitzt. Der nach Einengen der Reaktionslösung verbleibende Rückstand wird in Dichlormethan aufgenommen, mit Wasser gewaschen, die organische Phase über Natriumsulfat getrocknet und eingeengt. Zweifache Chromatographie an Kieselgel im Laufmittel Dichlormethan / Essigester 20:1 und Chromatograptue an Kieselgel im Laufmittel Petrolether / Essigester 3:1 ergibt 2,9 g (11% der Theorie) der Titelverbindung als Öl.

In Analogie zur Vorschrift des Beispiels 11 werden die in Tabelle 2 aufgeführten Beispiele hergestellt:

Tabelle 2:

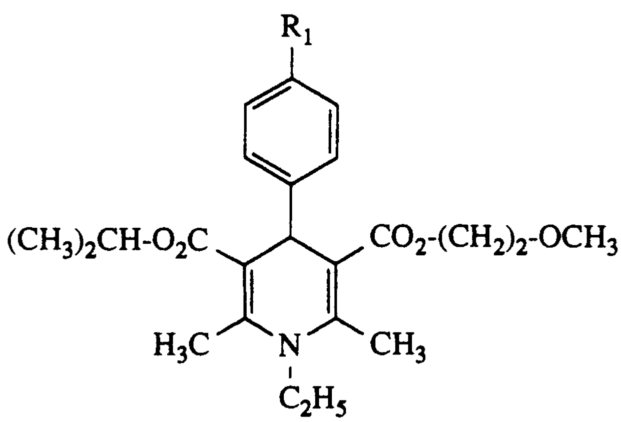

| Bsp.-Nr. | $R^1$ | Ausbeute (% d.Th.) | F°C |
|---|---|---|---|
| 12 | $-OCF_3$ | 21 | Racemat<br>49°C |
| 13 | $-CF_3$ | 12 | Racemat<br>Öl |

**Patentansprüche**

**Patentansprüche für folgende Vertragsstaaten : AT, CH, LI, BE, DE, DK, FR, GB, IT, LU, NL, PT, SE**

**1.** N-alkylierte 1,4-Dihydropyridmdicabonsäureester der allgemeinen Formel

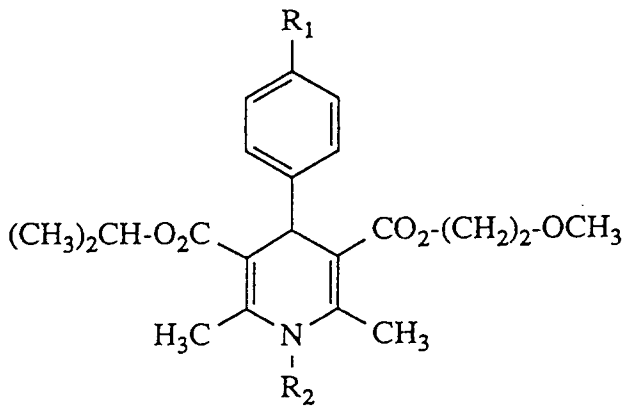

(I)

in welcher

$R^1$ für Trifluormethoxy, Cyano, Fluor, Chlor oder Trifluormethyl steht,

$R^2$ für Methyl, Ethyl oder Cyclopropyl steht,

mit der Maßgabe, daß $R^1$ nicht für Trifluormethyl stehen darf, wenn $R^2$ Methyl bedeutet.

**2.** N-alkylierte 1,4-Dihydropyridindicarbonsäureester der allgemeinen Formel I gemäß Anspruch 1 aus der Gruppe
1,2,6-Trimethyl-4-(4-trifluormethoxyphenyl)-1,4-dihydropyridin-3,5-dicarbonsäure-isopropyl-(2-methoxyethyl)-ester,
1,2,6-Trimethyl-4-(4-cyanophenyl)-1,4-dihydropyridin-3,5-dicarbonsäure-isopropyl-(2-methoxyethyl)-ester,
1-Ethyl-2,6-dimethyl-4-(4-trifluormethoxyphenyl)-1,4-dihydropyridin-3,5-dicarbonsäure-isopropyl-(2-methoxyethyl)-ester,
1,2,6-Trimethyl-4-(4-chlorphenyl)-1,4-dihydropyridin-3,5-dicarbonsäure-isopropyl-(2-methoxyethyl)-ester,
1,2,6-Trimethyl-4-(4-fluorphenyl)-1,4-dihydropyndin-3,5-dicarbonsäure-isopropyl-(2-methoxyethyl)-ester,
1-Ethyl-2,6-dimethyl-4-(4-trifluormethylphenyl)-1,4-dihydropyridin-3,5-dicarbonsäure-isopropyl-(2-methoxyethyl)-ester,
1-Cyclopropyl-2,6-dimethyl-4-(4-trifluormethoxyphenyl)-1,4-dihydropyridin-3,5-dicarbonsäure-isopropyl-(2-methoxyethyl)-ester,
in Form ihrer Racemate, (+)-sowie (-)-Isomeren.

**3.** N-alkylierte 1,4-Dihydropyridindicarbonsäureester der allgemeinen Formel I gemäß Anspruch 1 aus der Gruppe
(±)-1,2,6-Trimethyl-4-(4-trifluormethoxyphenyl)-1,4-dihydropyridin-3,5-dicarbonsäure-isopropyl-(2-methoxyethyl)-ester,
(+)-1,2,6-Trimethyl-4-(4-trifluormethoxyphenyl)-1,4-dihydropyridin-3,5-dicarbonsäure-isopropyl-(2-methoxyethyl)-ester,
(-)-1,2,6-Trimethyl-4-(4-trifluormethoxyphenyl)-1,4-dihydropyridin-3,5-dicarbonsäure-isopropyl-(2-methoxyethyl)-ester,
(±)-1,2,6-Trimethyl-4-(4-chlorphenyl)-1,4-dihydropyridin-3,5-dicarbonsäure-isopropyl-(2-methoxyethyl)-ester,
(+)-1,2,6-Trimethyl-4-(4-chlorphenyl)-1,4-dihydropyridin-3,5-dicarbonsäure-isopropyl-(2-methoxyethyl)-ester,
(-)-1,2,6-Trimethyl-4-(4-chlorphenyl)-1,4-dihydropyridin-3,5-dicarbonsäure-isopropyl-(2-methoxyethyl)-ester.

**4.** N-alkylierte 1,4-Dihydropyridincarbonsäureester nach Anspruch 1 bis 3 zur Behandlung von Krankheiten.

**5.** Verfahren zur Herstellung von N-alkylierten 1,4-Dihydropyridindicarbonsäureestern der allgemeinen Formel I gemäß Anspruch 1, dadurch gekennzeichnet, daß man

[A] Aldehyde der allgemeinen Formel

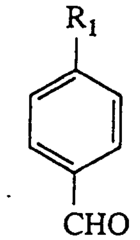

(II)

in welcher $R^1$ die oben angegebene Bedeutung hat,

entweder zuerst mit Acetessigsäureisopropylester der Formel (III) oder mit Acetessigsäure-2-methoxyethylester der Formel (IV)

$(CH_3)_2CH\text{-}O_2C\text{-}CH_2\text{-}CO\text{-}CH_3$ (III)

$H_3C\text{-}CO\text{-}CH_2\text{-}CO_2\text{-}(CH_2)_2\text{-}OCH_3$ (IV)

gegebenenfalls unter Isolierung, in die entsprechenden Ylidenverbindungen der allgemeinen Formel (V) oder (VI)

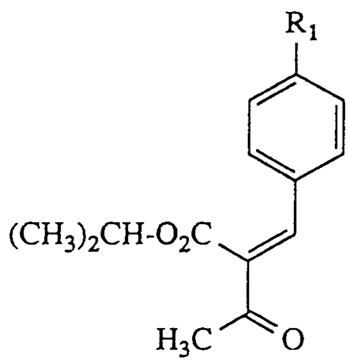

(V)

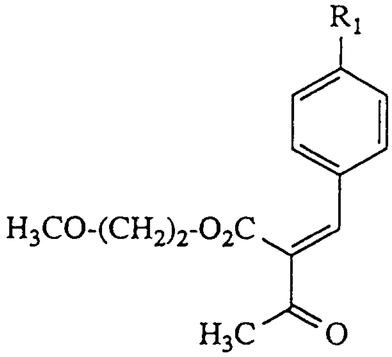

(VI)

in welcher

$R^1$ die oben angegebene Bedeutung hat,

überführt, anschließend im Fall der Verbindungen der allgemeinen Formel (V) mit Acetessigsäure-2-methoxyethylester der Formel (IV) und im Fall der Verbindungen der allgemeinen Formel (VI) mit Acetessigsäureisopropylester der Formel (III),

und mit Aminen oder den entsprechenden Aminhydrochloriden der allgemeinen Formel (VII)

$H_2N$-$R^2$ (VII)

in welcher

$R^2$ die oben angegebene Bedeutung hat,

in inerten Losemitteln umsetzt,

oder

[B] im Fall, daß $R^2$ für Methyl oder Ethyl steht,

zunächst die Verbindungen der allgemeinen Formel (V) mit Aminocrotonsäure-2-methoxyethylester der Formel (VIII)

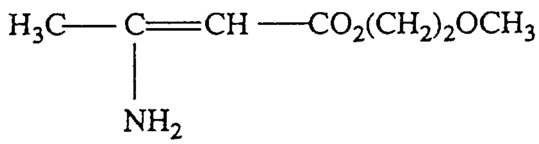

(VIII)

oder die Verbindungen der allgemeinen Formel (VI) mit β-Aminocrotonsäure-2-isopropylester der Formel (IX)

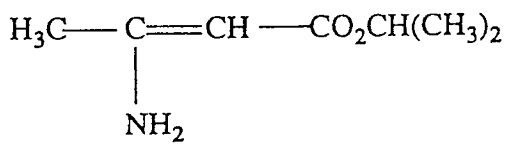

(IX)

in inerten Lösemitteln umsetzt und in einem letzten Schritt nach üblicher Methode, gegebenenfalls in Anwesenheit einer Base die -NH-Funktion alkyliert

und im Fall der enantiomerenreinen Ester entweder direkt eine chromatographische Trennung anschließt oder gegebenenfalls die jeweiligen enantiomerenreinen Carbonsäuren herstellt und diese, gegebenenfalls über ein reaktives Säurederivat, nach üblicher Methode verestert.

**6.** Arzneimittel enthaltend mindestens einen N-alkylierten 1,4-Dihydropyridindicarbonsäureester nach Anspruch 1 bis 3.

**7.** Verfahren zur Herstellung von Arzneimitteln nach Anspruch 6 dadurch gekennzeichnet, daß man den N-alkylierten 1,4-Dihydropyridindicarbonsäureester gegebenenfalls mit geeigneten Hilfs- und Trägerstoffen in eine geeignete Applikationsform überführt.

**8.** Verwendung der N-alkylierten 1,4-Dihydropyridindicarbonsäureester nach Anspruch 1 bis 3 zur Herstellung von Arzneimitteln.

**Patentansprüche für folgende Vertragsstaaten : ES, GR**

**1.** Verfahren zur Herstellung von N-alkylierten 1,4-Dihydropyridindicarbonsäureestern der allgemeinen Formel I

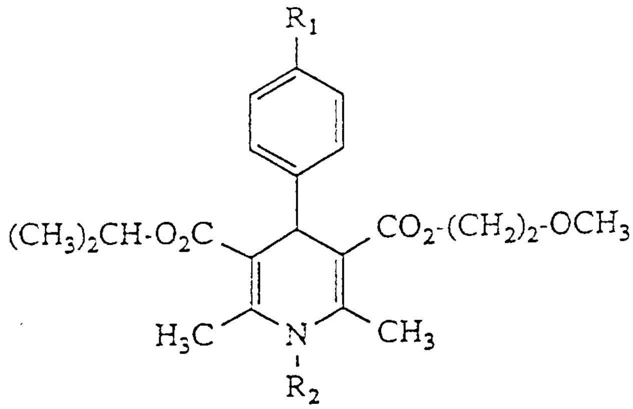

(I)

in welcher

$R^1$ für Trifluormethoxy, Cyano, Fluor, Chlor oder Trifluormethyl steht,

$R^2$ für Methyl, Ethyl oder Cyclopropyl steht,

mit der Maßgabe, daß $R^1$ nicht für Trifluormethyl stehen darf, wenn $R^2$ Methyl bedeutet, dadurch gekennzeichnet, daß man

[A] Aldehyde der allgemeinen Formel

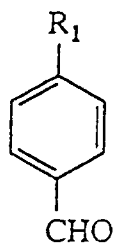

(II)

in welcher $R^1$ die oben angegebene Bedeutung hat,

entweder zuerst mit Acetessigsäureisopropylester der Formel (III) oder mit Acetessigsäure-2-methoxyethylester der Formel (IV)

$(CH_3)_2CH\text{-}O_2C\text{-}CH_2\text{-}CO\text{-}CH_3$ (III)

$H_3C\text{-}CO\text{-}CH_2\text{-}CO_2\text{-}(CH_2)_2\text{-}OCH_3$ (IV)

gegebenenfalls unter Isolierung, in die entsprechenden Ylidenverbindungen der allgemeinen Formel (V) oder (VI)

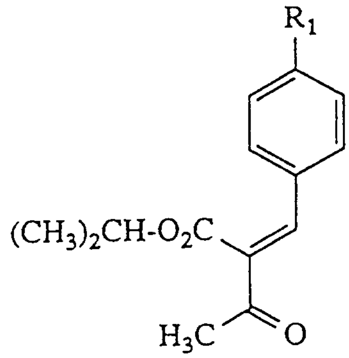

(V)

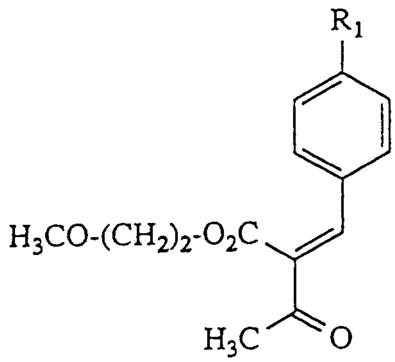

(VI)

in welcher

$R^1$ die oben angegebene Bedeutung hat,

überführt, anschließend im Fall der Verbindungen der allgemeinen Formel (V) mit Acetessigsäure-2-methoxyethylester der Formel (IV) und im Fall der Verbindungen der allgemeinen Formel (VI) mit Acetessigsäureisopropylester der Formel (III),

und mit Aminen oder den entsprechenden Aminhydrochloriden der allgemeinen Formel (VII)

$H_2N\text{-}R^2$ (VII)

in welcher

$R^2$ die oben angegebene Bedeutung hat,

in inerten Lösemitteln umsetzt,

oder

[B] im Fall, daß $R^2$ für Methyl oder Ethyl steht,

zunächst die Verbindungen der allgemeinen Formel (V) mit Aminocrotonsäure-2-methoxyethylester der Formel (VIII)

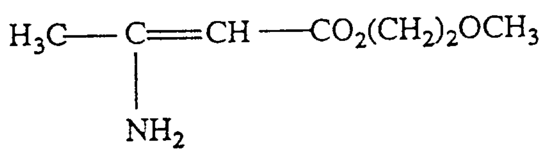

(VIII)

oder die Verbindungen der allgemeinen Formel (VI) mit $\beta$-Aminocrotonsäure-2-isopropylester der Formel (IX)

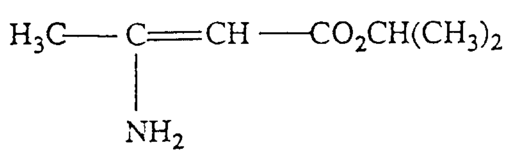

(IX)

in inerten Lösemitteln umsetzt und in einem letzten Schritt nach üblicher Methode, gegebenenfalls in Anwesenheit einer Base die -NH-Funktion alkyliert
und im Fall der enantiomerenreinen Ester entweder direkt eine chromatographische Trennung anschließt oder gegebenenfalls die jeweiligen enantiomerenreinen Carbonsäuren herstellt und diese, gegebenenfalls über ein reaktives Säurederivat, nach üblicher Methode verestert.

**2.** Verfahren zur Herstellung von Arzneimitteln nach Anspruch 1 dadurch gekennzeichnet, daß man den N-alkylierten 1,4-Dihydropyridindicarbonsäureester gegebenenfalls mit geeigneten Hilfs- und Trägerstoffen in eine geeignete Applikationsform überführt.

## Claims

## Claims for the following Contracting States : AT, BE, CH, DE, DK, FR, GB, IT, LI, LU, NL, PT, SE

**1.** N-Alkylated 1,4-dihydropyridinedicarboxylic acid esters of the general formula

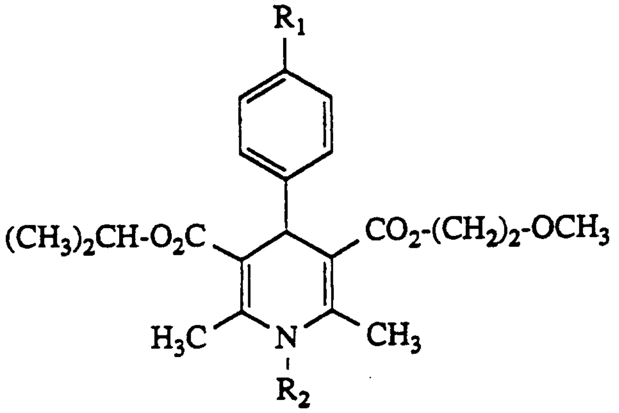

(I)

in which
$R^1$ represents trifluoromethoxy, cyano, fluorine, chlorine or trifluoromethyl,
$R^2$ represents methyl, ethyl or cyclopropyl,
with the proviso that $R^1$ must not represent trifluoromethyl if $R^2$ denotes methyl.

**2.** N-Alkylated 1,4-dihydropyridinedicarboxylic acid esters of the general formula I according to Claim 1 from the group consisting of
isopropyl 2-methoxyethyl 1,2,6-trimethyl-4-(4-trifluoromethoxyphenyl)-1,4-dihydropyridine3,5-dicarboxylate,
isopropyl 2-methoxyethyl 1,2,6-trimethyl-4-(4-cyanophenyl)-1,4-dihydropyridine-3,5-dicarboxylate,
isopropyl 2-methoxyethyl 1-ethyl-2,6-dimethyl-4-(4-trifluoromethoxyphenyl)-1,4-dihydropyridine-3,5-dicarboxylate,
isopropyl 2-methoxyethyl 1,2,6-trimethyl-4-(4-chlorophenyl)-1,4-dihydropyridine-3,5-dicarboxylate,
isopropyl 2-methoxyethyl 1,2,6-trimethyl-4-(4-fluorophenyl)-1,4-dihydropyridine-3,5-dicarboxylate,
isopropyl 2-methoxyethyl 1-ethyl-2,6-dimethyl-4-(4-trifluoromethylphenyl)-1,4-dihydropyridine-3,5-dicarboxylate,
isopropyl 2-methoxyethyl 1-cyclopropyl-2,6-dimethyl-4-(4-trifluoromethoxyphenyl)-1,4-dihydropyridine-3,5-dicarboxylate,
in the form of their racemates, (+)- or (-)-isomers.

**3.** N-Alkylated 1,4-dihydropyridinedicarboxylic acid esters of the general formula I according to Claim 1, from the group consisting of
isopropyl 2-methoxyethyl (±)-1,2,6-trimethyl-4-(4-trifluoromethoxyphenyl)-1,4-dihydropyridine-3,5-dicarboxylate,
isopropyl 2-methoxyethyl (+)-1,2,6-trimethyl-4-(4-trifluoromethoxyphenyl)-1,4-dihydropyridine-3,5-dicarboxylate,
isopropyl 2-methoxyethyl (-)-1,2,6-trimethyl-4-(4-trifluoromethoxyphenyl)-1,4-dihydropyridine-3,5-dicarboxylate,

isopropyl 2-methoxyethyl (±)-1,2,6-trimethyl-4-(4-chlorophenyl)-1,4-dihydropyridine-3,5-dicarboxylate, isopropyl 2-methoxyethyl ( + )-1,2,6-trimethyl-4-(4-chlorophenyl)-1,4-dihydropyridine-3,5-dicarboxylate, isopropyl 2-methoxyethyl (-)-1,2,6-trimethyl-4-(4-chlorophenyl)-1,4-dihydropyridine-3,5-dicarboxylate.

**4.** N-Alkylated 1,4-dihydropyridinedicarboxylic acid esters according to Claims 1 to 3 for the treatment of diseases.

**5.** Process for the preparation of N-alkylated 1,4-dihydropyridinedicarboxylic acid esters of the general formula I according to Claim 1,
characterised in that
[A] aldehydes of the general formula

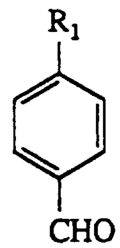

(II)

in which
$R^1$ has the abovementioned meaning,
are converted, first either with isopropyl acetoacetate of the formula (III) or with 2-methoxyethyl acetoacetate of the formula (IV)

$(CH_3)_2CH\text{-}O_2C\text{-}CH_2\text{-}CO\text{-}CH_3$ (III)

$H_3C\text{-}CO\text{-}CH_2\text{-}CO_2\text{-}(CH_2)_2\text{-}OCH_3$ (IV)

optionally with isolation, into the corresponding ylidene compounds of the general formula (V) or (VI)

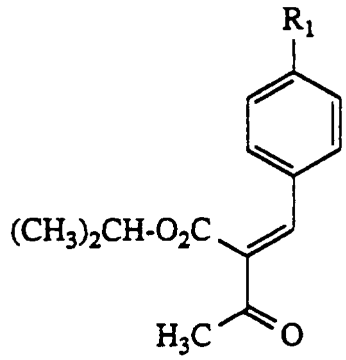

(V)

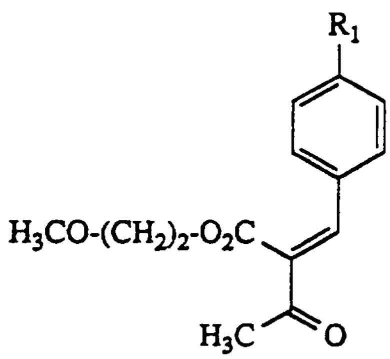

(VI)

in which

$R^1$ has the abovementioned meaning,

then in the case of the compounds of the general formula (V) these are reacted with 2-methoxyethyl acetoacetate of the formula (IV) and in the case of the compounds of the general formula (VI) these are reacted with isopropyl acetoacetate of the formula (III)

and with amines or the corresponding amine hydrochlorides of the general formula (VII)

$H_2N$-$R^2$ (VII)

in which

$R^2$ has the abovementioned meaning,

in inert solvents,

or

[B] in the case in which $R^2$ represents methyl or ethyl,

the compounds of the general formula (V) are first reacted with 2-methoxyethyl aminocrotonate of the formula (VIII)

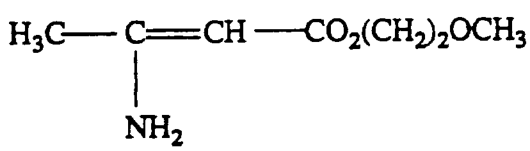

(VIII)

or the compounds of the general formula (VI) are first reacted with 2-isopropyl β-aminocrotonate of the formula (IX)

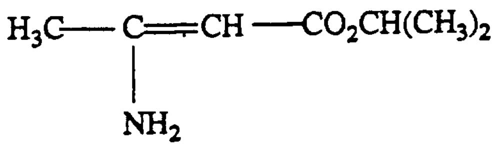

(IX)

in inert solvents and in a last step the -NH function is alkylated by a customary method, if appropriate in the presence of a base and in the case of the enantiomerically pure esters, either a chromatographic separation follows directly or the respective enantiomerically pure carboxylic acids are optionally prepared and the latter are esterified by a customary method, if appropriate via a reactive acid derivative.

**6.** Medicament containing at least one N-alkylated 1,4-dihydropyridinedicarboxylic acid ester according to Claims 1 to 3.

**7.** Process for the production of medicaments according to Claim 6, characterised in that the N-alkylated 1,4-dihydropyridinedicarboxylic acid esters are converted into a suitable administration form, if appropriate using suitable auxiliaries and excipients.

**8.** Use of the N-alkylated 1,4-dihydropyridine-dicarboxylic acid esters according to Claims 1 to 3 for the production of medicaments.

**Claims for the following Contracting States : ES, GR**

**1.** Process for the preparation of N-alkylated 1,4-dihydropyridinedicarboxylic acid esters of the general formula I

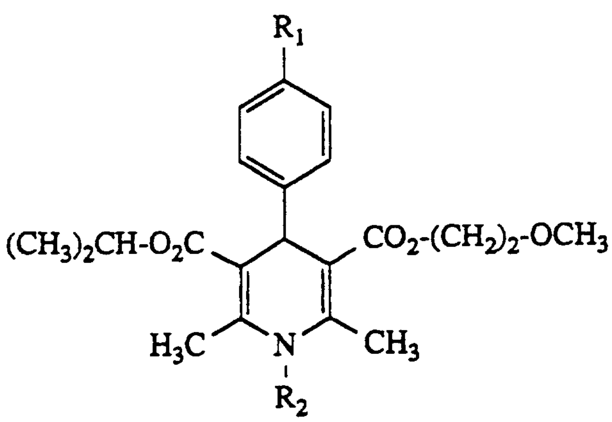

(I)

in which

$R^1$ represents trifluoromethoxy, cyano, fluorine, chlorine or trifluoromethyl,

$R^2$ represents methyl, ethyl or cyclopropyl,

with the proviso that $R^1$ must not represent trifluoromethyl if $R^2$ denotes methyl, characterised in that

[A] aldehydes of the general formula

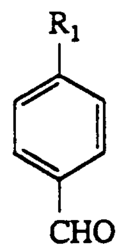

(II)

in which $R^1$ has the abovementioned meaning, are converted, first either with isopropyl acetoacetate of the formula (III) or with 2-methoxyethyl acetoacetate of the formula (IV)

$(CH_3)_2CH\text{-}O_2C\text{-}CH_2\text{-}CO\text{-}CH_3$ (III)

$H_3C\text{-}CO\text{-}CH_2\text{-}CO_2\text{-}(CH_2)_2\text{-}OCH_3$ (IV)

optionally with isolation, into the corresponding ylidene compounds of the general formula (V) or (VI)

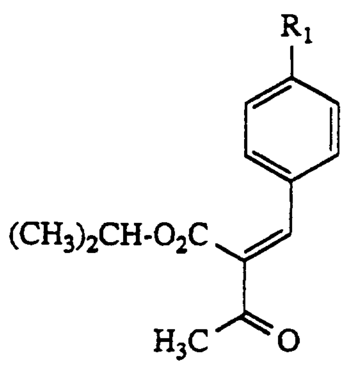

(V)

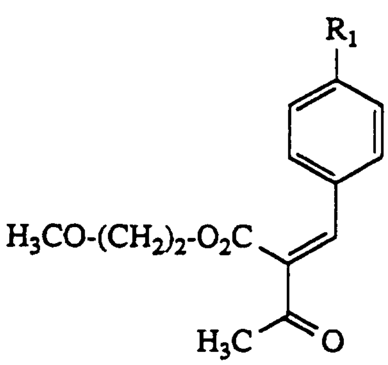

(VI)

in which

$R^1$ has the abovementioned meaning,

then in the case of the compounds of the general formula (V) these are reacted with 2-methoxyethyl acetoacetate of the formula (IV) and in the case of the compounds of the general formula (VI) these are reacted with isopropyl acetoacetate of the formula (III)

and with amines or the corresponding amine hydrochlorides of the general formula (VII)

$H_2N\text{-}R^2$ (VII)

in which

$R^2$ has the abovementioned meaning,

in inert solvents,

or

[B] in the case in which $R^2$ represents methyl or ethyl,

the compounds of the general formula (V) are first reacted with 2-methoxyethyl aminocrotonate of the formula (VIII)

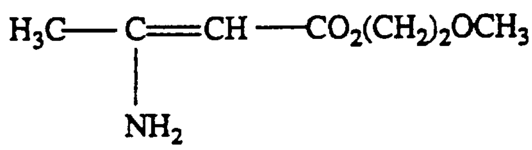

(VIII)

or the compounds of the general formula (VI) are first reacted with 2-isopropyl $\beta$-aminocrotonate of the formula (IX)

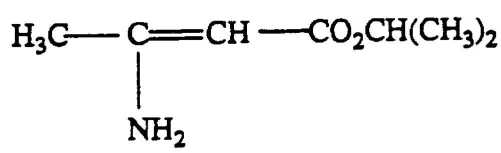

(IX)

in inert solvents and in a last step the -NH function is alkylated by a customary method, if appropriate in the presence of a base

and in the case of the enantiomerically pure esters, either a chromatographic separation follows directly or the respective enantiomerically pure carboxylic acids are optionally prepared and the latter are esterified by a customary method, if appropriate via a reactive acid derivative.

**2.** Process for the production of medicaments according to Claim 1, characterised in that the N-alkylated 1,4-dihydropyridinedicarboxylic acid esters are converted into a suitable administration form, if appropriate using suitable auxiliaries and excipients.

**Revendications**

**Revendications pour les Etats contractants suivants : AT, BE, CH, DE, DK, FR, GB, IT, LI, LU, NL, PT, SE**

**1.** Esters d'acides 1,4-dihydropyridine-dicarboxyliques N-alkylés de formule générale

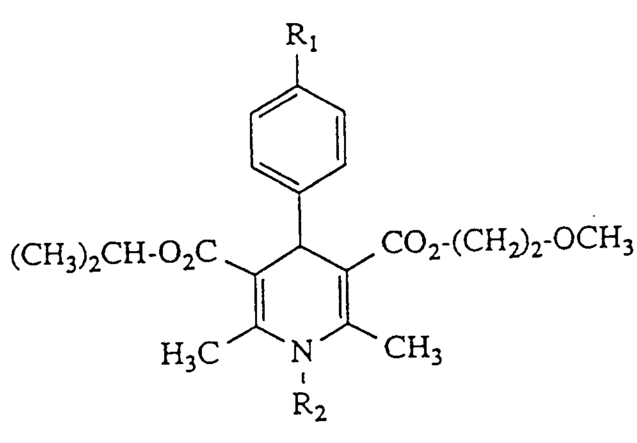

(I)

dans laquelle

$R^1$ est un groupe trifluorométhoxy, cyano, du fluor, du chlore ou un groupe trifluorométhyle,

$R^2$ est un groupe méthyle, éthyle ou cyclopropyle,

sous réserve que $R^1$ ne puisse pas représenter un groupe trifluorométhyle lorsque $R^2$ est un groupe méthyle.

**2.** Esters d'acides 1,4-dihydropyridine-dicarboxyliques N-alkylés de formule générale I suivant la revendication 1, du groupe :

ester d'isopropyle et de 2-méthoxyéthyle d'acide 1,2,6-triméthyl-4-(4-trifluorométhoxyphényl)-1,4-dihydropyridine-3,5-dicarboxylique,

ester d'isopropyle et de 2-méthoxyéthyle d'acide 1,2,6-triméthyl-4-(4-cyanophényl)-1,4-dihydropyridine-3,5-dicarboxylique,

ester d'isopropyle et de 2-méthoxyéthyle d'acide 1-éthyl-2,6-diméthyl-4-(4-trifluorométhoxyphényl)-1,4-dihydropyridine-3,5-dicarboxylique,

ester d'isopropyle et de 2-méthoxyéthyle d'acide 1,2,6-triméthyl-4-(4-chlorophényl)-1,4-dihydropyridine-3,5-dicarboxylique,

ester d'isopropyle et de 2-méthoxyéthyléthyle d'acide 1,2,6-triméthyl-4-(4-fluorophényl)-1,4-dihydropyridine-3,5-dicarboxylique,

ester d'isopropyle et de 2-méthoxyéthyle d'acide 1-éthyl-2,6-diméthyl-4-(4-trifluorométhylphényl)-1,4-dihydropyridine-3,5-dicarboxylique,

ester d'isopropyle et de 2-méthoxyéthyle d'acide 1-cyclopropyl-2,6-diméthyl-4-(4-trifluorométhoxyphényl)-1,4-dihydropyridine-3,5-dicarboxylique,

sous la forme de leurs racémates, de leurs isomères (+) ainsi que de leurs isomères (-).

**3.** Esters d'acides 1,4-dihydropyridine-dicarboxyliques N-alkylés de formule générale I suivant la revendication 1, du groupe :

ester d'isopropyle et de 2-méthoxyéthyle d'acide (±)-1,2,6-triméthyl-4-(4-trifluorométhoxyphényl)-1,4-dihydropyridine-3,5-dicarboxylique,

ester d'isopropyle et de 2-méthoxyéthyle d'acide (+)-1,2,6-triméthyl-4-(4-trifluorométhoxyphényl)-1,4-dihydropyridine-3,5-dicarboxylique,

ester d'isopropyle et de 2-méthoxyéthyle d'acide (-)-1,2,6-triméthyl-4-(4-trifluorométhoxyphényl)-1,4-dihydropyridine-3,5-dicarboxylique,

ester d'isopropyle et de 2-méthoxyéthyle d'acide (±)-1,2,6-triméthyl-4-(4-chlorophényl)-1,4-dihydropyridine-3,5-dicarboxylique,

ester d'isopropyle et de 2-méthoxyéthyle d'acide (+)-1,2,6-triméthyl-4-(4-chlorophényl)-1,4-dihydropyridine-3,5-dicarboxylique,

ester d'isopropyle et de 2-méthoxyéthyle d'acide (-)-1,2,6-triméthyl-4-(4-chlorophényl)-1,4-dihydropyridine-3,5-dicarboxylique.

**4.** Esters d'acides 1,4-dihydropyridine-dicarboxyliques N-alkylés suivant les revendications 1 à 3, destinés à combattre des maladies.

**5.** Procédé de production d'esters d'acides 1,4-dihydropyridine-dicarboxyliques N-alkylés de formule I suivant la revendication 1, caractérisé en ce que

[A] on transforme des aldéhydes de formule générale

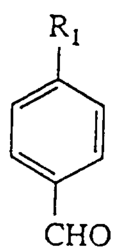

(II)

dans laquelle $R^1$ a la définition indiquée ci-dessus,

tout d'abord avec l'ester isopropylique d'acide acétylacétique de formule (III) ou avec l'ester 2-méthoxyéthylique d'acide acétylacétique de formule (IV)

$(CH_3)_2CH\text{-}O_2C\text{-}CH_2\text{-}CO\text{-}CH_3$ (III)

$H_3C\text{-}CO\text{-}CH_2\text{-}CO_2\text{-}(CH_2)_2\text{-}OCH_3$ (IV)

en les composés ylidéniques correspondants de formule générale (V) ou (VI)

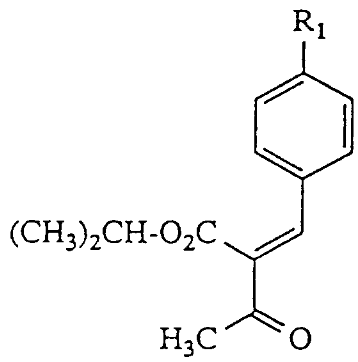
(V)

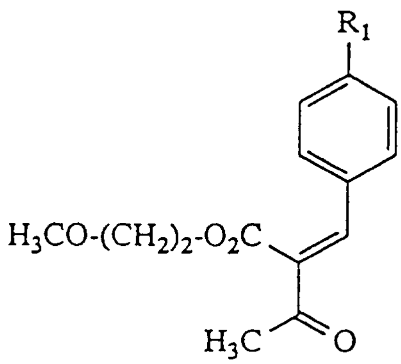
(VI)

dans laquelle
$R^1$ a la définition indiquée ci-dessus,
en isolant éventuellement ces composés, puis, dans le cas des composés de formule générale (V), on fait réagir ces composés avec l'ester 2-méthoxyéthylique de l'acide acétylacétique de formule (IV) et, dans le cas des composés de formule générale (VI), on fait réagir les composés avec l'ester isopropylique de l'acide acétylacétique de formule (III),
et avec des amines ou les chlorhydrates d'amines correspondants de formule générale (VII)

$H_2N\text{-}R^2$ (VII)

dans laquelle
$R^2$ a la définition indiquée ci-dessus,
dans des solvants inertes,
ou bien
[B] au cas où $R^2$ est un groupe méthyle ou éthyle, on fait tout d'abord réagir les composés de formule générale (V) avec l'ester 2-méthoxyéthylique de l'acide aminocrotonique de formule (VIII)

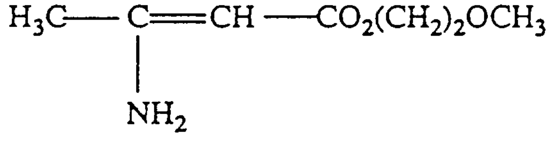
(VIII)

ou les composés de formule générale (VI) avec l'ester 2-isopropylique de l'acide $\beta$-aminocrotonique

de formule (IX)

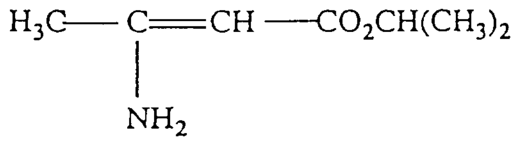

(IX)

dans des solvants inertes et dans une dernière étape, on alkyle la fonction -NH par un procédé classique, éventuellement en présence d'une base,

et dans le cas des esters de pureté énantiomérique, on effectue ensuite directement une séparation chromatographique ou bien, le cas échéant, on prépare les acides carboxyliques de pureté énantiomérique correspondants et on les estérifie par un procédé classique, en passant le cas échéant par un dérivé d'acide réactif.

**6.** Médicament contenant au moins un ester d'acide 1,4-dihydropyridine-dicarboxylique N-alkylé suivant les revendications 1 à 3.

**7.** Procédé de préparation de médicaments suivant la revendication 6, caractérisé en ce qu'on fait prendre une forme administrable appropriée aux esters d'acides 1,4-dihydropyridine-dicarboxyliques N-alkylés, le cas échéant avec des substances auxiliaires et des supports appropriés.

**8.** Utilisation des esters d'acides 1,4-dihydropyridine-dicarboxyliques N-alkylés suivant les revendications 1 à 3 pour la préparation de médicaments.

**Revendications pour les Etats contractants suivants : ES, GR**

**1.** Procédé de production d'esters d'acides 1,4-dihydropyridine-dicarboxyliques N-alkylés de formule générale I

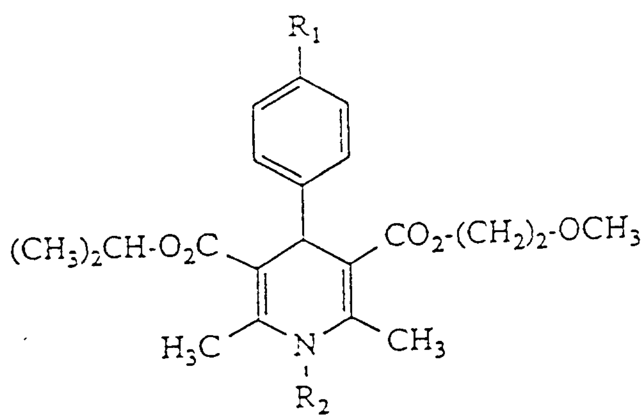

(I)

dans laquelle

$R^1$ est un groupe trifluorométhoxy, cyano, du fluor, du chlore ou un groupe trifluorométhyle,

$R^2$ est un groupe méthyle, éthyle ou cyclopropyle,

sous réserve que $R^1$ ne puisse pas représenter un groupe trifluorométhyle lorsque $R^2$ est un groupe méthyle,

caractérisé en ce que

[A] on transforme les aldéhydes de formule générale

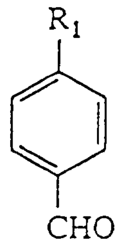

(II)

dans laquelle $R^1$ a la définition indiquée ci-dessus,

tout d'abord avec l'ester isopropylique d'acide acétylacétique de formule (III) ou avec l'ester 2-méthoxyéthylique d'acide acétylacétique de formule (IV)

$(CH_3)_2CH\text{-}O_2C\text{-}CH_2\text{-}CO\text{-}CH_3$ (III)

$H_3C\text{-}CO\text{-}CH_2\text{-}CO_2\text{-}(CH_2)_2\text{-}OCH_3$ (IV)

en les composés ylidéniques correspondants de formule générale (V) ou (VI)

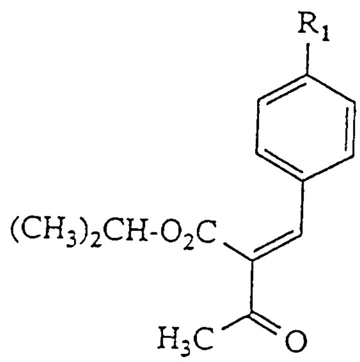

(V)

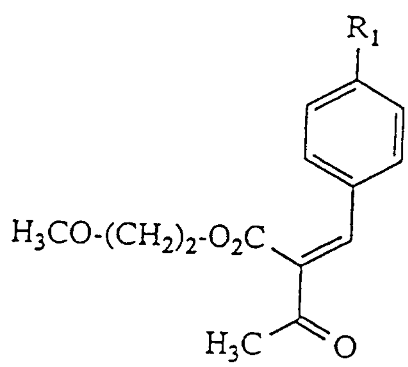

(VI)

dans laquelle

$R^1$ a la définition indiquée ci-dessus,

en isolant éventuellement ces composés, puis, dans le cas des composés de formule générale (V), on fait réagir ces composés avec l'ester 2-méthoxyéthylique de l'acide acétylacétique de formule (IV) et, dans le cas des composés de formule générale (VI), on fait réagir les composés avec l'ester isopropylique de l'acide acétylacétique de formule (III),

et avec des amines ou les chlorhydrates d'amines correspondants de formule générale (VII)

$H_2N\text{-}R^2$ (VII)

dans laquelle

$R^2$ a la définition indiquée ci-dessus,

dans des solvants inertes,

ou bien

[B] au cas où $R^2$ est un groupe méthyle ou éthyle, on fait tout d'abord réagir les composés de formule générale (V) avec l'ester 2-méthoxyéthylique de l'acide aminocrotonique de formule (VIII)

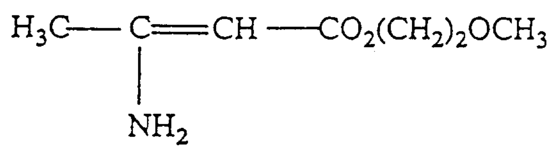

(VIII)

ou les composés de formule générale (VI) avec l'ester 2-isopropylique de l'acide $\beta$-aminocrotonique de formule (IX)

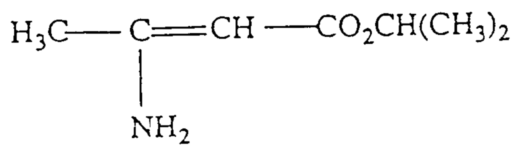

(IX)

dans des solvants inertes et dans une dernière étape, on alkyle la fonction -NH par un procédé classique, éventuellement en présence d'une base,

et dans le cas des esters de pureté énantiomérique, on effectue ensuite directement une séparation chromatographique ou bien, le cas échéant, on prépare les acides carboxyliques de pureté énantiomérique correspondants et on les estérifie par un procédé classique, en passant le cas échéant par un dérivé d'acide réactif.

**2.** Procédé de préparation de médicaments suivant la revendication 1, caractérisé en ce qu'on fait prendre une forme administrable appropriée à des esters d'acides 1,4-dihydropyridine-dicarboxyliques N-alkylés, le cas échéant avec des substances auxiliaires et des supports appropriés.